(19) Europäisches Patentamt
European Patent Office
Office européen des brevets

(11) Veröffentlichungsnummer: **0 050 275**
**B1**

(12) # EUROPÄISCHE PATENTSCHRIFT

(45) Veröffentlichungstag der Patentschrift: **19.10.83**

(21) Anmeldenummer: **81108075.3**

(22) Anmeldetag: **08.10.81**

(51) Int. Cl.³: **C 07 C 127/24,** C 07 C 125/063, C 08 G 18/10, C 08 G 18/83, C 08 G 18/50

(54) **Verfahren zur Herstellung von Polyaminen.**

(30) Priorität: **21.10.80 DE 3039600**

(43) Veröffentlichungstag der Anmeldung: **28.04.82 Patentblatt 82/17**

(45) Bekanntmachung des Hinweises auf die Patenterteilung: **19.10.83 Patentblatt 83/42**

(84) Benannte Vertragsstaaten: **AT BE CH DE FR GB IT LI NL**

(56) Entgegenhaltungen:
**ANALYST, Band 104, Oktober 1979, LONDON (GB) R. F. WALKER et al.: »Spectrophotometric Determination of Aliphatic Isocyanates in the Occupational Atmosphere; Part 1. Determination of Total Isocyanate Concentration«, Seiten 928–936.**

(73) Patentinhaber: **BAYER AG, Zentralbereich Patente, Marken und Lizenzen, D-5090 Leverkusen 1, Bayerwerk (DE)**

(72) Erfinder: **Rasshofer, Werner, Dr., Wolfskaul 10, D-5000 Koeln 80 (DE)**
Erfinder: **Dieterich, Dieter, Dr., Ludwig-Girtler-Strasse 1, D-5090 Leverkusen (DE)**
Erfinder: **Meyborg, Holger, Dr., Bergisch-Gladbacher Strasse 1, D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Verfahren zur Herstellung von Polyaminen

Die Erfindung betrifft ein verbessertes Verfahren zur Herstellung von Polyaminen, welche Urethan- und/oder Harnstoffgruppen und/oder Biuretgruppen sowie vorzugsweise auch Ethergruppen enthalten, durch alkalische Hydrolyse von endständigen Isocyanatgruppen aufweisenden Vorpolymeren.

Es ist bekannt, daß aromatische Isocyanate durch saure Hydrolyse in primäre Amine überführt werden können. Die Reaktion verläuft allerdings nur sehr unvollständig, da das bei der Hydrolyse gebildete Amin mit noch nicht umgesetztem Isocyanat zum entsprechenden Harnstoff weiter reagiert. Diese Folgereaktion läßt sich auch durch Anwendung überschüssiger starker Mineralsäure nicht unterdrücken. Ein neueres Beispiel findet sich in der JP-PS 55 007-827.

In der DE-AS 1 270 046 wird ein Verfahren zur Herstellung definierter, Polyalkylenglykolether-Segmente enthaltender, primärer aromatischer Amine beschrieben, bei dem man Umsetzungsprodukte von aromatischen Di- oder Triisocyanaten mit Polyalkylenglykolethern und/oder Polyalkylenglykolthioethern, vorzugsweise solchen mit Molekulargewichten zwischen 400 und 4000, mit sekundären oder tertiären Carbinolen umsetzt und anschließend (gegebenenfalls in Gegenwart saurer Katalysatoren) in einem inerten Lösungsmittel einer thermischen Spaltung unterwirft. Nachteilig ist dabei, daß im Verlauf der thermischen Spaltung der Urethane brennbare, leicht flüchtige Alkene entstehen, die im Gemisch mit Luft explosiv sind, so daß entsprechende Vorsichtsmaßnahmen getroffen werden müssen.

Gegenstand der DE-AS 1 694 152 ist die Herstellung von mindestens zwei endständigen Aminogruppen aufweisenden Präpolymeren durch Umsetzung von Hydrazin, Aminophenylethylamin oder anderen Diaminen mit einem NCO-Präpolymer aus einem Polyetherpolyol und Polyisocyanat (NCO: NH-Verhältnis=1 : 1,5 bis 1 : 5). Nicht-umgesetztes Amin muß dabei in einem weiteren Schritt sorgfältig entfernt werden, da es die Umsetzung mit Polyisocyanaten stark katalysiert, so zu kurzen Verarbeitungszeiten führt und auch selbst als Reaktionspartner auftritt.

Eine andere Synthesemöglichkeit für Urethangruppen aufweisende Polyamine wird in der FR-PS 1 415 317 beschrieben. Urethangruppenhaltige NCO-Präpolymere werden mit Ameisensäure in die N-Formylderivate überführt, die zu endständigen aromatischen Aminen verseift werden. Auch die Reaktion von NCO-Präpolymeren mit Sulfaminsäure gemäß DE-AS 1 555 907 führt zu Verbindungen mit endständigen Aminogruppen. Des weiteren werden höhermolekulare, aliphatische, sekundäre und primäre Aminogruppen aufweisende Voraddukte nach DE-AS 1 215 373 durch Umsetzung höhermolekularer Hydroxylverbindungen mit Ammoniak in Gegenwart von Katalysatoren unter Druck bei hohen Temperaturen erhalten oder gemäß US-PS 3 044 989 durch Umsetzung höhermolekularer Polyhydroxylverbindungen mit Acrylnitril unter anschließender katalytischer Hydrierung. Auch durch Umsetzung von NCO-Präpolymeren mit Hydroxylgruppen aufweisenden Enaminen, Aldiminen oder Ketiminen und anschließende Hydrolyse erhält man gemäß DE-OS 2 546 536 bzw. US-PS 3 865 791 höhermolekulare, endständige Aminogruppen und Urethangruppen aufweisende Verbindungen.

Überraschenderweise wurde gefunden, daß man definierte, Urethan- und/oder Harnstoff- und/oder Biuretgruppen enthaltende aromatische oder aliphatische primäre Amine direkt erhält, wenn man entsprechende NCO-Prepolymere mit geringfügig überschüssigen wäßrigen oder wäßrige Lösungsmittel enthaltenden Basenlösungen vermischt, z. B. Alkalilaugen eintropft, und die entstandenen (Alkali)Carbamate mit saurem Ionenaustauscher behandelt.

Gegenstand der Erfindung ist somit ein Verfahren zur Herstellung von Urethan- und/oder Harnstoff- und/oder Biuretgruppen aufweisenden Polyaminen durch Hydrolyse von endständigen Isocyanatgruppen aufweisenden Verbindungen, welches dadurch gekennzeichnet ist, daß man

1. in einem ersten Schritt ein Urethan- und/oder Harnstoff- und/oder Biuretgruppen aufweisendes NCO-Präpolymer, gegebenenfalls gelöst in einem mit Wasser mischbaren inerten organischen Lösungsmittel, gegebenenfalls unter Kühlung auf Temperaturen von 0—40°C bevorzugt 10 bis 20°C, durch Vermischen mit wäßrigen und/oder wäßrige Lösungsmittel enthaltenden Basenlösungen in das Carbamat überführt, wobei das Äquivalentverhältnis zwischen Hydroxid- und NCO-Gruppen 0,3 : 1 bis 1,3 : 1 ist und vorzugsweise zwischen 1,01 : 1 und 1,30 : 1 liegt,
2. das Carbamat in einem zweiten Schritt durch Umsatz mit einer mindestens äquivalenten Menge eines sauren Ionenaustauschers unter $CO_2$-Abspaltung direkt in das freie Amin überführt, das
3. nach an sich bekannter Weise abgetrennt wird.

Verbindungen, welche durch die allgemeine Formel

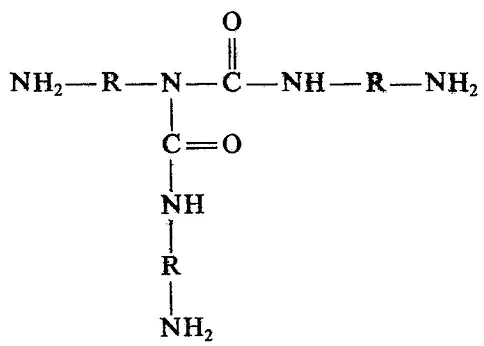

in der

R gleiche oder verschiedene Reste, die zweiwertige, geradkettige oder verzweigte aliphatische Reste, vorzugsweise den Tetra-, Hexa-, Octa-, Deca-, Undeca- und Dodeca-methylenrest, bevorzugt den Hexamethylenrest,
zweiwertige aliphatische Reste, wie den 1,3- und/oder 1,4-Cyclohexanrest, den 4,4'- und/oder 2,4'-Dicyclohexylmethanrest oder seine Mono-, Di-, Tri- und Tetra-$C_1-C_4$-alkylsubstituierten Derivate, den 2,4- und/oder 2,6-Methylcyclohexanrest und den Isophoronrest

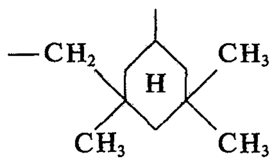

vorzugsweise den 4,4'- und/oder 2,4'-Dicyclohexylmethanrest, den 2,4- und/oder 2,6-Methylcyclohexanrest und besonders den Isophoronrest,

bedeuten, charakterisiert werden können und können aus den zugrundeliegenden, an sich bekannten Biurettriisocyanaten hergestellt werden. Diese Biurettriisocyanate enthalten für gewöhnlich noch kleinere Anteile (etwa <10 Gew.-%) an höherfunktionellen Biuretpolyisocyanaten, welche 2 oder mehrere Biuretgruppen in abnehmenden Mengen enthalten.

Entsprechend enthalten die daraus hergestellten Polyamine auch in kleineren, abnehmenden Anteilen höherfunktionelle Amine mit zwei und mehr Biuretgruppen.

Die nach dem erfindungsgemäßen Verfahren zugänglichen Polyamine können mit Urethan- und/oder Harnstoff- und/oder Biuret-Gruppen zur Herstellung von gegebenenfalls zellförmigen Polyurethankunststoffen und Polyurethanschaumstoffen verwendet werden durch Umsetzung von

A) Polyisocyanaten mit
B) Polyaminen, sowie gegebenenfalls
C) weiteren niedermolekularen und/oder höhermolekularen Verbindungen mit gegenüber Isocyanaten reaktiven Gruppen, gegebenenfalls
D) in Anwesenheit an sich bekannter Hilfs- und Zusatzstoffe,

wobei als Komponente B) die nach dem erfindungsgemäßen Verfahren hergestellten Polyamine eingesetzt werden.

Gegenüber herkömmlichen Verfahren besitzt das vorliegende Verfahren folgende überraschende Vorteile:

1. Bei der Herstellung der NCO-Präpolymeren aus Polyisocyanaten und NCO-aktive H-Atome enthaltenden Verbindungen bleiben oftmals geringe Anteile an monomerem Isocyanat im Präpolymer. Es hat sich gezeigt, daß im erfindungsgemäßen Verfahren nur die entsprechenden niedermolekularen Polyamine mit dem Ionenaustauscher Assoziate ergeben und mit diesen aus dem Reaktionsgemisch entfernt werden können. Überraschenderweise geschieht das jedoch nicht mit den höhermolekularen Polyaminen, so daß nach dem vorliegenden Verfahren höhermolekulare Polyamine direkt und nicht über dem Umweg mineralsaurer Salze, sowie mit erheblich verbesserter Molekular-Einheitlichkeit zugänglich sind.
2. Es ist gleichermaßen möglich, den Ionenaustauscher zur Carbamatlösung bzw. -suspension zu geben oder das umgekehrte Verfahren zu wählen.
3. Das erfindungsgemäße Verfahren ist sehr schonend und führt dazu, daß die Polyamine, auch solche auf aromatischer Basis, allgemein als farblose bis leicht gelbe Produkte anfallen und durch

die Abwesenheit von Verunreinigungen wesentlich langsamer oxidieren.

4. Mit dem erfindungsgemäßen Verfahren ist eine so schonende Verfahrensweise aufgefunden worden, daß auch die Herstellung von empfindlichen biuretgruppenhaltigen Polyaminen ohne Schwierigkeiten gelingt und somit eine neue Verbindungsklasse von Biuretpolyaminen nach dem Verfahren zugänglich wird.
5. Es ist möglich, mehr als die äquivalente Menge an saurem Ionenaustauscher, bezogen auf die H-Atome, zu verwenden, ohne daß die höhermolekularen Polyamine in die entsprechenden Salze, was einen weiteren Verfahrensschritt unter Anfall von unerwünschtem Salzballast erfordern würde, übergeführt werden.

Das im erfindungsgemäßen Verfahren eingesetzte NCO-Präpolymer wird in an sich bekannter Weise durch Umsetzung von Wasser und/oder hoch- und/oder niedermolekularen, Hydroxy- und/oder Amino- und/oder Thiolgruppen enthaltenden Verbindungen (Molekulargewicht: 60 bis ca. 12 000) mit einem Überschuß an Polyisocyanat hergestellt. Es kommen hierfür im Prinzip beliebige, von hydrolysierbaren Gruppen (abgesehen von den NCO-Gruppen) freie, aliphatische, cycloaliphatische, araliphatische, aromatische und heterocyclische Polyisocyanate, wie sie z. B. von W. Siefken in Justus Liebigs Annalen der Chemie, 562, Seiten 75 bis 136, beschrieben werden, beispielsweise solche der Formel

$$Q\,(NCO)_n$$

in der

n 2 bis 4, vorzugsweise 2, und
Q einen aliphatischen Kohlenwasserstoffrest mit 2 bis 18, vorzugsweise 6 bis 10 C-Atomen, einen cycloaliphatischen Kohlenwasserstoffrest mit 4 bis 15, vorzugsweise 5 bis 10 C-Atomen, einen aromatischen Kohlenwasserstoffrest mit 6 bis 15, vorzugsweise 6 bis 13 C-Atomen, oder einen araliphatischen Kohlenwasserstoffrest mit 8 bis 15, vorzugsweise 8 bis 13 C-Atomen,

bedeuten, z. B. 1,4-Tetramethylendiisocyanat, 1,6-Hexamethylendiisocyanat, 1,12-Dodecandiisocyanat, ferner cycloaliphatische Diisocyanate in beliebigen Mischungen ihrer Stereoisomeren, z. B. Cyclobutan-1,3-diisocyanat, Cyclohexan-1,3- und -1,4-diisocyanat, 1-Isocyanato-3,3,5-trimethyl-5-isocyanatomethyl-cyclohexan, 2,4- und 2,6-Hexahydrotoluylendiisocyanat, Hexahydro-1,3- und/oder -1,4-phenylendiisocyanat, Perhydro-2,4'- und/oder -4,4'-diphenylmethandiisocyanat; insbesondere sind jedoch aromatische Diisocyanate geeignet, z. B. 1,3- und 1,4-Phenylendiisocyanat, 2,4- und 2,6-Toluylendiisocyanat, sowie beliebige Gemische dieser Isomeren, Diphenylmethan-2,4'- und/oder -4,4'-diisocyanat, einschließlich seiner Alkyl- und Chlor-substituierten Derivate und Naphthylen-1,5-diisocyanat.

Ferner kommen beispielsweise in Frage: Triphenylmethan-4,4',4''-triisocyanat, Polyphenyl-polymethylen-polyisocyanate, wie sie durch Anilin-Formaldehyd-Kondensation und anschließende Phosgenierung erhalten und z. B. in den GB-PS 874 430 und 848 671 beschrieben werden, m- und p-Isocyanatophenylsulfonyl-isocyanate gemäß der US-PS 3 454 606, perchlorierte Arylpolyisocyanate, wie sie z. B. in der DE-Auslegeschrift 1 157 601 (US-PS 3 277 138) beschrieben werden, Norbornan-Diisocyanate gemäß US-PS 3 492 330, Isocyanuratgruppen aufweisende Polyisocyanate, wie sie z. B. in der US-PS 3 001 973, in den DE-PS 1 022 789, 1 222 067 und 1 027 394 sowie in den DE-PS 1 929 034 und 2 004 048 beschrieben werden, Urethangruppen aufweisende Polyisocyanate, wie sie z. B. in der BE-PS 752 261 oder in den US-PS 3 394 164 und 3 644 457 beschrieben werden, acylierte Harnstoffgruppen aufweisende Polyisocyanate gemäß der DE-PS 1 230 778 sowie durch Telomerisationsreaktionen hergestellte Polyisocyanate, wie sie z. B. in der US-PS 3 654 196 beschrieben werden.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Besonders bevorzugt werden in der Regel die technisch leicht zugänglichen Polyisocyanate, z. B. das 2,4- und 2,6-Toluylendiisocyanat sowie beliebige Gemische dieser Isomeren (»TDI«), Polyphenyl-polymethylen-polyisocyanate, wie sie durch Anilin-Formaldehyd-Kondensation und anschließende Phosgenierung hergestellt werden (»rohes MDI«), sowie Urethangruppen, Isocyanuratgruppen oder Harnstoffgruppen aufweisende Polyisocyanate (»modifizierte Polyisocyanate«), insbesondere solche modifizierten Polyisocyanate, die sich vom 2,4- und/oder 2,6-Toluylendiisocyanat bzw. vom 4,4'- und/oder 2,4'-Diphenylmethandiisocyanat ableiten.

Für die Herstellung des NCO-Präpolymeren werden vorzugsweise Verbindungen mit einem Molekulargewicht von 400 bis 12 000, insbesondere 400 bis 5000, eingesetzt, welche mindestens 2, vorzugsweise 2 bis 4, insbesondere 2 oder 3, Hydroxyl-, Amino- und/oder Thiolgruppen (bevorzugt Hydroxylgruppen) aufweisen und frei an leicht hydrolysierbaren Gruppen wie z. B. Estergruppen sind. In Frage kommen beispielsweise die in der Polyurethanchemie üblichen Polyacetale, Polythioether

und insbesondere Polyether.

Die erfindungsgemäß in Frage kommenden, mindestens zwei, in der Regel zwei bis acht, vorzugsweise zwei bis drei, Hydroxylgruppen aufweisenden Polyether sind solche der an sich bekannten Art und werden z. B. durch Polymerisation von Epoxiden wie Ethylenoxid, Propylenoxid, Butylenoxid, Tetrahydrofuran, Styroloxid oder Epichlorhydrin mit sich selbst, z. B. in Gegenwart von Lewis-Katalysatoren wie $BF_3$, oder durch Anlagerung dieser Epoxide, vorzugsweise von Ethylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z. B. Ethylenglykol, Propylenglykol-(1,3) oder -(1,2), Trimethylolpropan, Glycerin, Sorbit, 4,4'-Dihydroxy-diphenylpropan, Anilin, Ethanolamin oder Ethylendiamin hergestellt. Auch Sucrosepolyether, wie sie z. B. in den DE-AS 1 176 358 und 1 064 938 beschrieben werden, sowie auf Formit oder Formose gestartete Polyether (DE-OS 2 639 083 bzw. 2 737 951), kommen erfindungsgemäß in Frage. Vielfach sind solche Polyether bevorzugt, die überwiegend (bis zu 90 Gew.-%, bezogen auf alle vorhandenen OH-Gruppen im Polyether) primäre OH-Gruppen aufweisen. Auch OH-Gruppen aufweisende Polybutadiene sind erfindungsgemäß geeignet.

Unter den Polythioethern seien insbesondere die Kondensationsprodukte von Thiodiglykol mit sich selbst und/oder mit anderen Glykolen angeführt.

Auch bereits Urethan- oder Harnstoffgruppen enthaltende Polyhydroxylverbindungen sowie gegebenenfalls modifizierte natürliche Polyole sind verwendbar. Auch Anlagerungsprodukte von Alkylenoxiden an Phenol- Formaldehyd-Harze oder auch an Harnstoff-Formaldehydharze sind erfindungsgemäß einsetzbar.

Die genannten Polyhydroxylverbindungen können vor ihrer Verwendung noch in der verschiedensten Weise modifiziert werden: So läßt sich gemäß DE-OS 2 210 839 (US-PS 3 849 515) und 2 544 195 ein Gemisch aus verschiedenen Polyhydroxylverbindungen durch Veretherung in Gegenwart einer starken Säure zu einem höhermolekularen Polyol kondensieren, welches aus über Etherbrücken verbundenen verschiedenen Segmenten aufgebaut ist. Es ist auch möglich, z. B. gemäß DE-OS 2 559 372 in die Polyhydroxylverbindungen Amidgruppen einzuführen.

Vertreter der genannten erfindungsgemäß zu verwendenden Verbindungen sind z. B. in High Polymers, Vol. XVI, »Polyurethanes, Chemistry and Technology«, verfaßt von Saunders-Frisch, Interscience Publishers, New York, London, Band I, 1962, Seiten 32 bis 42 und Seiten 44 bis 54 und Band II, 1964, Seiten 5 und 6 und 198 und 199, sowie im Kunststoffhandbuch, Band VII, Vieweg—Höchtlen, Carl-Hanser-Verlag, München, 1966, z. B. auf den Seiten 45 bis 71, beschrieben. Selbstverständlich können Mischungen der obengenannten Verbindungen mit mindestens zwei gegenüber Isocyanaten reaktionsfähigen Wasserstoffatomen mit einem Molekulargewicht von 400 bis 12 000, z. B. Mischungen von verschiedenen Polyethern, eingesetzt werden.

Für die Herstellung des im erfindungsgemäßen Verfahren einzusetzenden NCO-Präpolymeren kommen,gegebenenfalls nur anteilsweise, als Ausgangskomponenten auch Verbindungen mit mindestens zwei gegenüber Isocyanaten reaktionsfähigen Wasserstoffatomen und einem Molekulargewicht von 18 bis 400, vorzugsweise 60 bis 400, in Betracht. Auch in diesem Fall versteht man hierunter Wasser und/oder Hydroxylgruppen und/oder Aminogruppen und/oder Thiolgruppen, vorzugsweise Hydroxylgruppen aufweisende Verbindungen, wie sie als Kettenverlängerungsmittel oder Vernetzungsmittel aus der Polyurethanchemie an sich bekannt sind. Diese Verbindungen weisen in der Regel 2 bis 8, vorzugsweise 2 bis 4, gegenüber Isocyanaten reaktionsfähige Wasserstoffatome auf.

Auch in diesem Fall können Mischungen von verschiedenen Verbindungen mit mindestens zwei gegenüber Isocyanaten reaktionsfähigen Wasserstoffatomen mit einem Molekulargewicht von 18 bis 400 verwendet werden.

Als Beispiele für derartige Verbindungen seien genannt: Wasser, Ethylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bis-hydroxymethyl-cyclohexan, 2-Methyl-1,3-propandiol, Dibrombutendiol (US-PS 3 723 392), Glyzerin, Trimethylolpropan, Hexantriol-(1,2,6), Trimethylolethan, Pentaerythrit, Chinit, Mannit und Sorbit, Ricinusöl, Di-, Tri- und Tetraethylenglykol, Di-, Tri- und Tetrapropylenglykol, Dibutylenglykol und höhere Polyethylen-, Polypropylen- oder Polybutylenglykole mit einem Molekulargewicht bis 400, 4,4'-Dihydroxy-diphenylpropan, Di-hydroxymethyl-hydrochinon, Ethanolamin, Diethanolamin, N-Methyldiethanolamin, Triethanolamin und 3-Aminopropanol.

Als niedermolekulare Polyole kommen auch die Gemische von Hydroxyaldehyden und Hydroxyketonen (»Formose«) bzw. die hieraus durch Reduktion erhaltenen mehrwertigen Alkohole (»Formit«) in Frage, wie sie bei der Selbstkondensation von Formaldehydhydrat in Gegenwart von Metallverbindungen als Katalysator und von zur Endiolbildung befähigten Verbindungen als Co-Katalysator entstehen (DE-OS 2 639 084, 2 714 084, 2 714 104, 2 271 186, 2 738 154 und 2 738 512).

Erfindungsgemäß geeignete aliphatische Diamine sind beispielsweise Ethylendiamin, 1,4-Tetramethylendiamin, 1,6-Hexamethylendiamin, 1,12-Dodecamethylendiamin sowie deren Gemische, 1-Amino-3,3,5-trimethyl-5-aminomethylcyclohexan (»Isophorondiamin«), 2,4- und 2,6-Hexahydrotoluylendiamin sowie deren Gemische, Perhydro-2,4'- und 4,4'-diaminodiphenylmethan, p-Xylylendiamin, Bis-(3-aminopropyl)-methylamin, Diamino-perhydroanthrazene (DE-OS 2 638 731) und cycloaliphatische Triamine gemäß DE-OS 2 614 244. Auch Hydrazin und substituierte Hydrazine, z. B.

Methylhydrazin, kommen erfindungsgemäß in Betracht.

Als Beispiele für aromatische Diamine seien die Ethergruppen aufweisenden Diamine gemäß DE-OS 1 770 525 und 1 809 172 (US-PS 3 654 364 und 3 736 295), gegebenenfalls in 5-Stellung substituierte 2-Halogen-1,3-phenylendiamine (DE-OS 2 001 772, 2 025 896 und 2 065 869), 3,3'-Dichlor-4,4'-diamino-diphenylmethan, Toluylendiamin, 4,4'-Diaminodiphenylmethan, 4,4'-Diaminodiphenyldisulfide (DE-OS 2 404 976), Diaminodiphenyldithioether (DE-OS 2 509 404), durch Alkylthiogruppen substituierte aromatische Diamine (DE-OS 2 638 760), Sulfonat- oder Carboxylatgruppen enthaltende aromatische Diamine (DE-OS 2 720 166) sowie die in der DE-OS 2 635 400 aufgeführten hochschmelzenden Diamine genannt. Beispiele für aliphatisch-aromatische Diamine sind die Aminoalkylthioaniline gemäß DE-OS 2 734 574.

Die freie Isocyanatgruppen aufweisenden Präpolymeren werden in an sich bekannter Weise durch Umsetzung der Reaktionspartner in der Schmelze oder in Lösung hergestellt. Das Äquivalentverhältnis von NCO-Gruppen zu aktiven Wasserstoffatomen (bevorzugt OH-Gruppen) ist in jedem Fall größer als 1, soll in der Regel zwischen 1,5 : 1 und 2,8 : 1 liegen. Selbstverständlich ist es möglich, einen noch größeren Überschuß an Polyisocyanat zu verwenden. Die Voraddukte haben je nach den gewählten Ausgangskomponenten im allgemeinen ölige bis wachsartige Konsistenz. Beträgt das NCO/OH-Verhältnis mehr als 2, so erhält man im wesentlichen nicht verlängerte Voraddukte, während NCO/OH-Verhältnisse von unter 2 eine Erhöhung des mittleren Molekulargewichts der Voraddukte zur Folge haben. Es ist, wie schon erläutert, auch möglich, niedermolekulare Polyole als Kettenverlängerungsmittel bei der Herstellung der Präpolymere anteilsmäßig neben höhermolekularen Ausgangsverbindungen mitzuverwenden; in diesem Fall erhält man ebenfalls höhermolekulare Voraddukte.

Für das erfindungsgemäße Verfahren werden Präpolymere bevorzugt, die aus höhermolekularen Polyetherglykolen, gegebenenfalls unter Mitverwendung von Kettenverlängerern der oben beschriebenen Art, und aliphatischen und/oder aromatischen Diisocyanaten im Äquivalentverhältnis von 1 : 1,5 bis 1 : 2,8, insbesondere ca. 1 : 2, erhalten worden sind.

Gleichfalls bevorzugt sind NCO-Gruppen-haltige Harnstoffe, wie sie durch Reaktion von NCO-Verbindungen mit Wasser oder mit aromatischen bzw. aliphatischen Aminen entstehen.

Besonders bevorzugt sind NCO-Gruppen-haltige Biurete, wie sie durch Reaktion von NCO-Verbindungen mit einem Unterschuß Wasser oder durch Reaktion von Harnstoffen mit NCO-Verbindungen zustande kommen.

Der NCO-Gehalt der NCO-Verbindungen, die erfindungsgemäß in die Polycarbamate bzw. Polyamine überführt werden, liegt zwischen etwa 0,5 Gew.-% NCO und 37,5 Gew.-% NCO, vorzugsweise 0,7 und 27 Gew.-%.

Als saure Ionenaustauscher im erfindungsgemäßen Verfahren sind alle Stoffe geeignet, die in einem polymeren unlöslichen Grundgerüst bewegliche acide Wasserstoffatome besitzen.

Besonders geeignet als polymere Säuren sind im erfindungsgemäßen Verfahren Ionenaustauscherharze, die als polymere Basis ein Styrol/Divinylbenzol-Skelett besitzen, an das Sulfonsäure-Gruppen als saure Funktionen angeheftet sind.

Im erfindungsgemäßen Verfahren wird das Präpolymer zunächst in der Regel in einem mit Wasser mischbaren, inerten Lösungsmittel gelöst. Geeignete Solventien sind z. B. Dimethoxyethan (DME), Tetrahydrofuran oder Dioxan. Dabei können auf 100 Teile Lösungsmittel z. B. 1 bis 400 Teile des Präpolymeren verwendet werden. Das Präpolymer wird zweckmäßig unter Rühren langsam (vorzugsweise binnen 30 bis 120 Minuten) mit einer vorzugsweise auf ca. 0 bis 40°C temperierten Lösung von Alkali- oder Erdalkalihydroxiden oder anderen starken Basen, wie Tetralkylammoniumhydroxiden oder Alkalialuminaten vermischt (z. B. eingetropft in die Base), wobei die Basenkonzentration vorzugsweise 1 Gew.-Teil Base auf 5 bis 20 Gew.-Teile Wasser bzw. Alkohol beträgt. Gleichfalls geeignet sind anorganische und organische Ammoniumhydroxide (z. B. Tetraalkylammoniumhydroxid).

Wird ohne Solvens gearbeitet, so wird das möglichst niedrigviskose (bevorzugt bis ca. 5000 mPa s), s), gegebenenfalls vorher erwärmte (z. B. 30—90°C) NCO-Präpolymer bei hoher Rührgeschwindigkeit möglichst fein verteilt zugegeben (z. B. Einspritzung durch eine Düse) und zur Rührerleichterung die vorgelegte Wassermenge gegebenenfalls erhöht (z. B. um den Faktor 1,1—100).

Das NCO-Präpolymer oder seine Lösung kann auch gemeinsam mit der Base in einen Reaktionsraum (z. B. Staticmischer, Rührkammermischer) eindosiert werden und in einem Verweilgefäß bis zur Beendigung der Reaktion verbleiben.

Die Basen-, vorzugsweise (Erd)Alkalihydroxidmenge wird bevorzugt so bemessen, daß nach vollständiger Reaktion noch eine geringe Menge freier Base verbleibt; ein NCO/OH-Ionen-Verhältnis von 1 : 1,01 bis 1 : 1,30 und die Verwendung von Alkalihydroxiden wird dabei bevorzugt. Die Restbasenkonzentration darf nicht zu hoch sein, da sonst nach der Bildung des Carbamats im Präpolymer enthaltende Urethangruppierungen gleichfalls hydrolysiert werden. Um die Homogenität der Lösungen zu verbessern, wird vorzugsweise ein handelsüblicher Emulgator in Mengen von 0,1 bis 1 Gew.-Teilen, bevorzugt ca. 0,5 Gew.-Teile (bezogen auf 100 Teile Reaktionsgemisch) zugesetzt. Intensives Rühren ist bei der Vermischung der NCO-Komponente mit der Hydroxidkomponente zu empfehlen, um lokale Konzentrationsungleichgewichte zu vermeiden. Nach der Beendigung der

Zugabe des Präpolymeren wird vorzugsweise noch ca. 15 bis 180 Minuten bei 0 bis 20° C weitergerührt. Es ist jedoch auch möglich, ein NCO-OH-Ionen-Verhältnis unterschüssiger Base, z. B. von 1 : 0,3 bis 1 : 0,99 einzusetzen, wobei dann der Anteil an über Harnstoffgruppen »vorverlängerten«, höheres Molekulargewicht und höhere Viskosität aufweisenden Polycarbamaten bzw. Polyaminen zunimmt.

Im zweiten Schritt wird die Carbamat-Lösung bzw. Carbamat-Suspension mit dem Ionenaustauscher vereinigt. Für das erfindungsgemäße Verfahren ist es unwesentlich, ob zur vorgelegten Carbamatsuspension bzw. Carbamatlösung das saure Ionenaustauscherharz zugegeben (Verfahren A) oder ob umgekehrt vorgegangen wird (Verfahren B). Ionenaustauscherharz und Carbamat-Komponente werden in dem Maße miteinander vereinigt, wie es die Heftigkeit der Gasentwicklung und die Dimensionierung der Apparatur zuläßt (10 bis 300 Minuten).

Diese Gasentwicklung tritt jedoch erst nach der Zugabe von ca. 1/4 der Gesamtmenge an Ionenaustauscherharz ein.

Bei der Vereinigung bei den Komponenten tritt Temperaturerhöhung auf, ein gegebenenfalls durch externes Heizen einzustellender Temperaturbereich von 10 bis 70° C erwies sich als günstig.

Es wird so lange Ionenaustauscher zugesetzt, bis keine Gasentwicklung zu erkennen ist. Kurzzeitiges Erwärmen auf 60 bis 100° C treibt gelöstes Kohlendioxid aus.

Das umgekehrte Verfahren — Ionenaustauscher vorgelegt — ist v. a. dann zu bevorzugen, wenn die Reaktionsdaten bekannt sind (Verfahren B), sowie bei kontinuierlicher Arbeitsweise. Am Endpunkt der Reaktion ist das Reaktionsgemisch basisch, wie es dem Gehalt an freiem Amin und dessen Basenstärke entspricht.

Die einfache Aufarbeitung gestaltet sich wie folgt:

1. Ist das Amin löslich, so wird abfiltriert und die aminische Lösung vom Lösungsmittel durch Destillation befreit.
2. Ist das Amin schwerlöslich, so wird ein geeignetes Solvens bis zur Lösung des Amins zugegeben und wie oben verfahren.
3. Ist das Amin unlöslich, so wird vom flüssigen Medium abfiltriert, der Rückstand mit einem geeigneten Solvens bis zur Lösung des Amins behandelt und wie oben verfahren.

Geeignete Solventien sind aprotisch-dipolare wassermischbare Lösungsmittel wie N-Methylpyrrolidon, Dimethylformamid, Dimethylsulfoxid etc.

Die beladenen Ionenaustauscherharze (neutrale Form) lassen sich nach bekannten Methoden regenerieren und erneut problemlos einsetzen. Alle so erhaltenen Polyamine werden z. B. bei 0,013 bis 0,13 mbar/60 bis 80° C von Spuren flüchtiger Bestandteile befreit.

Die erfindungsgemäß erhaltenen Polyamine werden wegen ihres niedrigen Dampfdrucks vorzugsweise als Reaktionspartner für Polyisocyanate bei der Herstellung von gegebenenfalls zelligen Polyurethankunststoffen eingesetzt, wobei sie gegebenenfalls auch mit anderen niedermolekularen (Molekulargewicht: 32 bis 399) und/oder höhermolekularen (Molekulargewicht: 400 bis ca. 15 000) Verbindungen mit gegenüber Isocyanaten reaktiven Gruppen kombiniert werden können. Geeignete Ausgangskomponenten für die Herstellung von Polyurethankunststoffen werden oben im Zusammenhang mit der Präpolymerherstellung bzw. auch in DE-OS 2 302 564, DE-OS 2 432 764 (US-PS 3 963 679) sowie in den DE-OS 2 639 083, 2 512 385, 2 513 815, 2 550 796, 2 550 797, 2 550 833, 2 550 860 und 2 550 862 genannt. Dort finden sich auch Hinweise auf bei der Polyurethanherstellung gegebenenfalls mitzuverwendende Hilfs- und Zusatzstoffe.

Weitere Verwendungszwecke der erfindungsgemäß hergestellten Polyamine sind z. B. Kupplungskomponenten für Diazofarbstoffe, Härter für Epoxid- und Phenolharze sowie alle anderen an sich bekannten Reaktionen von Aminen wie Amid- oder Imidbildung etc.

Die folgenden Beispiele dienen der Erläuterung des erfindungsgemäßen Verfahrens. Wenn nicht anders vermerkt, sind Mengenangaben als Gewichtsteile bzw. Gewichtsprozente zu verstehen.

## Beispiel 1

### Herstellung des Carbamats

Zu einer Lösung von 67,2 g (1,2 Mol) KOH und 0,1 g Mersolat®H in 200 ml Wasser wird eine Lösung von 200 g einer durch Biuretisierung von 1,6-Diisocyanatohexan gewonnenen Verbindung (enthält 1,12 Mol NCO, NCO-Wert = 23) in 200 ml Dioxan bei 10 bis 15° C in 30 Minuten zugetropft, wobei externe Kühlung nötig ist. 30 Minuten wird bei dieser Temperatur nachgerührt.

### Herstellung des Amins (Verfahren A)

680 g feuchtes Lewatit®SC 108 werden innerhalb 150 Minuten in wenigen Portionen hinzugegeben, wobei eine Temperatur von 60 bis 70° C eingehalten wird. Dabei werden 20 l $CO_2$ gemessen. Bei stark

nachlassender Gasentwicklung wird mit 1 l Dioxan versetzt und 10 Minuten auf 90°C erhitzt. Dabei werden nochmals 5,1 l $CO_2$ gemessen, wodurch sich eine Gesamtgasausbeute von 25,1 l = 98,8% der Theorie, ergibt. Die heiße Mischung wird abgesaugt, wobei 80 g (46,8%) eines harzartigen, nur gering gelb gefärbten Produkts mit korrektem $^1H$—NMR-Spektrum erhalten werden.

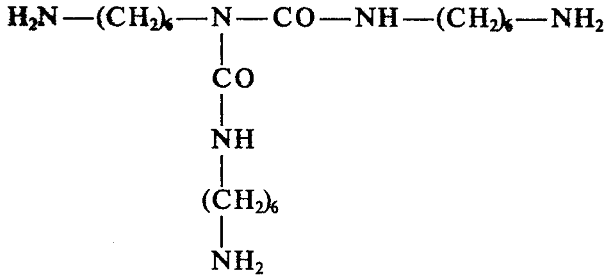

Lewatit®SC 108 (Bayer)

Ein stark saurer Kationenaustauscher mit $SO_3H$-Gruppen in einer gelförmigen Divinylbenzol/Styrol-Matrix (8% vernetzt mit einem Kornspektrum von 0,3 bis 1,5 mm). Die Totalkapazität beträgt 2 mval/1 ml feuchtes Harz bzw. 4,5 mval/1 g trockenes Harz. Die nutzbaren Kapazitäten sind ca. 1,3 mval/ml resp. 3 mval/g.

Es wurden in gleichartiger Durchführungsform folgende Ionenaustauscherharze im Verfahren des Beispiels 1 eingesetzt, wobei das Polyamin in vergleichbarer Ausbeute und Reinheit erhalten wurde:

1) Lewatit®S 100 (Bayer):

Ein stark saurer Kationenaustauscher mit —$SO_3H$-Gruppen in einer Styrol/Divinylbenzol-Matrix. Die Totalkapazität beträgt 2,2 mval/ml feuchtes Harz, die nutzbare Kapazität bis 1,8 mval/ml (Schüttgewicht 800 bis 900 g/l feuchtes Harz).

2) Lewatit® SP 112 (Bayer):

Ein stark saurer Kationenaustauscher mit $SO_3H$-Gruppen und mittlerer Vernetzung in einer makroporös feinporigen Styrol/Divinylbenzol-Matrix. Die Totalkapazität beträgt ca. 1,9 mval/ml feuchtes Harz, die nutzbare Kapazität bis 1,3 mval/ml (Schüttgewicht 700 bis 800 g/l feuchtes Harz).

3) Lewatit® SP 120 (Bayer):

Ein stark saurer Kationenaustauscher mit $SO_3H$-Gruppen und hohem Vernetzungsgrad in einer makroporös großporigen Styrol/Divinylbenzol-Matrix. Die Totalkapazität beträgt 1,9 mval/ml feuchtes Harz, die nutzbare Kapazität bis 30 g CaO/l feuchtes Harz (Schüttgewicht 700 bis 800 g/l feuchtes Harz).

4) Lewasorb® A 10 (Bayer):

Es handelt sich hierbei um eine auf eine Korngröße von <0,2 mm vermahlenen Austauscher vom Lewatit 100-Typ (H-Form).

Weitere Angaben zu den verwendeten Ionenaustauscherharzen finden sich in den Schriften der BAYER AG: OC/I 1379, OC/I 1350, OC/I 20 026, OC/I 20 031, OC/I 20 303 und OC/I 20 333.

Sämtliche verwendeten Ionenaustauscher wurden vorher in die $H^{\oplus}$-Form überführt und mit destilliertem Wasser säurefrei gewaschen.

Vergleichsbeispiel

Wird obiges Carbamat mit Mineralsäure behandelt und das entstandene Salz mit KOH neutralisiert, so erhält man bei nur ca. 30 bis 50%iger $CO_2$-Gasausbeute ein festes Produkt, das zu einem großen Teil

unlöslich ist (Harnstoffbildung).

Beispiel 2

Verfahren A

Herstellung des NCO-Präpolymers

1 kg eines auf Propylenglykol gestarteten Polypropylendiols mit einer OH-Zahl von 56, einem mittleren Molekulargewicht von 2000 und einem Anteil primärer OH-Gruppen von <5% wird durch 5stündiges Erhitzen auf 80° C bei 20 mbar entwässert.

Dieses Polyol wird innerhalb 5 Stunden bei 80° C zu 174 g (1 Mol) 2,6-Diisocyanatotoluol getropft und 90 Minuten bei 80° C nachgerührt. Das NCO-Präpolymer hat einen NCO-Wert von 3,3% (Theorie 3,58). In weiteren Ansätzen wurden Präpolymere mit den NCO-Werten 4,92% (entspricht 2,79% freiem 2,6-Diisocyanatotoluol) und 3,4% erhalten.

Herstellung des Carbamats

9,6 g (172 mmol) Kaliumhydroxid und 0,1 g Mersolat® H werden in 50 ml Wasser gelöst. Innerhalb einer Stunde wird eine Lösung von 200 g des obigen Präpolymers (NCO 3,3%) in 200 ml trockenem Dioxan bei 10° C dazugetropft. Zur Vervollständigung der Reaktion wird 15 Minuten weitergerührt.

Herstellung des Amins

Zu dieser Carbamatsuspension werden mit einem Pulverdosiertrichter binnen 1 Stunde 84 g Lewasorb® A 10 zugesetzt. Die Temperatur beträgt 20 bis 40° C. Es entwickeln sich 3,05 l $CO_2$ (86,6% der Theorie). Das Lewasorb® A 10 wird abgesaugt und mit 350 ml Dioxan gewaschen. Die dioxanischen Phasen werden vereinigt und das Dioxan abdestilliert.

Es hinterbleiben 178 g einer schwach-gelblichen Flüssigkeit (91,5% Ausb.), die folgende Daten zeigte:

prim. Basen-Stickstoff: 0,82
Molmasse: 2580 (th. 2296)
NH-Wert: 41 (20° C), 59 (60° C), 53 (100° C)
theoretischer NH-Wert bei einer Molmasse von 2580: 43,4.

Wird aus 5246 g des obigen Präpolymers (gelöst in 4 l trockenem Dioxan) mit einem NCO-Wert von 3,4, 280 g KOH und 3 g Mersolat® H in zusammen 1 l Wasser ein Carbamat hergestellt und dies mit 4012 g Lewatit® SP 120 (H-Form, wasserfeucht) versetzt (4 Stunden Raumtemperatur, 2 Stunden 50° C) und im übrigen wie oben verfahren, so wird ein Amin mit folgenden Daten erhalten:

Ausbeute: 4400 g leicht gefärbtes Öl (85,7% der Theorie)
Molmasse: 2420 (th. 2296)
NH-Wert: 39,4
theoretischer NH-Wert bei einer Molmasse von 2420: 46.

Verfahren B

NCO-Präpolymer (3,3% NCO) und Carbamat werden wie oben hergestellt.

Herstellung des Amins

Es werden 150 g Lewatit® 5100 und 100 ml Wasser vorgelegt. Bei Raumtemperatur werden innerhalb 20 Minuten 200 g des obigen Carbamats zugetropft und 2 Stunden bei 40 bis 45° C nachgerührt. Die Gasausbeute betrug 3,1 l (88,6% der Theorie). Das Lewatit wurde abgetrennt und das Solvens abdestilliert.

Es hinterbleiben 140 g (72% der Theorie) einer fast farblosen, viskosen Flüssigkeit mit den folgenden Daten:

Molmasse: 2380, 2330 (th. 2296) (dampfdruckosmometrisch bestimmt)

Titration mit 0,1 n $HClO_4$: 762 ml 0,1 n $HClO_4$ für 1 g Substanz, das entspricht 2,0 Mol $NH_2$ bei einem Molekulargewicht von 2620 (aus Titration) (vgl. dazu den theoretischen Wert- aus dem Ansatz berechnet: 1,75 Mol $NH_2$ bei einem Molekulargewicht von 2296; bzw. den theoretischen Wert aus der dampfdruckosmometrischen Messung).

Beispiel 3

Präpolymer

Ein Addukt von etwa 3 Mol Diisocyanatotoluol und 1 Mol Trimethylolpropan wurde verwendet (NCO-Wert des Triisocyanats 16,2, th. 18,8).

Carbamat

44,8 g (0,8 Mol) KOH und 0,2 g Mersolat® H werden in 150 ml Wasser gelöst und mit einer Lösung von 200 g des Triisocyanates in 200 ml trockenem Dioxan bei 5 bis 10°C binnen 30 Minuten versetzt. 30 Minuten wird bei dieser Temperatur weitergerührt.

Amin (nach Verfahren A)

Zu dieser Carbamatsuspension wurden innerhalb 195 Minuten ein Gemisch von 654 g Lewatit® SP 120 (H-Form, wasserfeucht) und 393 g Lewatit® SP 112 bei einer Reaktionstemperatur von 60 bis 65°C portionsweise zugegeben. Es wird die Entwicklung von 13,9 l $CO_2$ (80,3% der Theorie) beobachtet. Nachdem durch Filtration der Feststoff von der Flüssigkeit abgetrennt wurde, wird das Amin mit 1,8 l heißem Dioxan aus dem Ionenaustauscher herausgelöst. Nach dem abermaligen Filtrieren wird das Dioxan abdestilliert. Es resultieren 146 g (81% der Theorie) eines gelblich gefärbten Produktes mit einem Schmelzbereich von 95 bis 115° C. Die Daten lauten:

Molmasse: 960 (th. nach dem NCO gef.-Wert des Desmodur L: 702)
NH-Wert (20° C): 159, 160 (th. bei einer Molmasse von 960 : 175).

Beispiel 4

Herstellung eines Integralschaums

Rezeptur A:

1. 100 g des nach Beispiel 2 erhaltenen Amins
2. 14 g Butan-1,4-diol
3. 1 g Ethylenglykol
4. 8 g Trichlormonofluormethan
5. 0,02 g Dibutylzinndilaurat
6. 0,3 g Triethylendiamin
7. 77 g ein durch Umsetzung mit unterschüssigen Mengen an Dipropylenglykol modifiziertes, flüssiges Diphenylmethan-4,4'-diisocyanat mit einem NCO-Gehalt von 22 Gew.-%.

Rezeptur B:

1. 100 g des nach Beispiel 2 erhaltenen Amins
2. 30 g Ethylenglykol
3. 8 g Trichlorfluormethan
4. 0,02 g Dibutylzinndilaurat
5. 0,2 g Triethylendiamin
6. 190 g des in Rezeptur A/7 genannten flüssigen Diisocyanates.

Die Komponenten 1 bis 6 (Rezeptur A) bzw. 1 bis 5 (Rezeptur B) werden bei 25°C mit einem Schnellrührer 30 Sekunden intensiv miteinander vermischt. Dann wird die Isocyanatkomponente hinzugegeben, weitere 10 Sekunden mit einem Schnellrührer intensiv vermischt und in einem Freischaumpäckchen verschäumt.

Es wurden folgende Daten erhalten:

$T_1$ = Startzeit (Zeit in Sekunden nach der Einrührung der Isocyanatkomponente, bei der das Gemisch zu schäumen beginnt);
$T_2$ = Steigzeit (Zeit in Sekunden nach der Einrührung der Isocyanatkomponente, nach der der Steigvorgang beendet ist);
$T_3$ = Klebfreizeit (Zeit in Sekunden nach der Einrührung der Isocyanatkomponente, nach der die Oberfläche des Schaums nicht mehr klebrig ist);
$T_4$ = Zupfzeit (Zeit in Sekunden nach der Einrührung der Isocyanatkomponente, nach der es nicht mehr möglich ist, kleine Stücke mit den Fingern aus dem Schaum zu reißen.

| | $T_1$ | $T_2$ | $T_3$ | $T_4$ |
|---|---|---|---|---|
| Rezeptur 1 | 12 | 33 | 33 | 40 |
| Rezeptur 2 | 22 | 36 | 36 | 36 |

Das nach Rezeptur 1 erhaltene Kunststoffmaterial zeigt nur geringen Schrumpf und weist leicht elastisches Verhalten auf.

Das nach Rezeptur 2 erhaltene Kunststoffmaterial ist wesentlich härter als der PU-Kunststoff nach Rezeptur 1 und zeigt keinerlei Schrumpf.

## Beispiel 5

### Herstellung eines Weichschaums

Rezeptur:

1. 100 g des nach Beispiel 2 erhaltenen Amins
2. 3 g Wasser
3. 0,5 g eines Schaumstabilisators auf der Basis eines Polysiloxan-Polyalkylenoxid-Blockcopolymerisats
4. 0,1 g Diethylaminoethanol
5. 0,2 g N-Methyl-N'-dimethylamino-piperazin
6. 0,05 g Zinn-II-di-(2-ethyl-hexanoat)
7. 36,0 g Toluylendiisocyanat (80% 2,4- und 20% 2,6-Isomeres).

Die Komponenten 1 bis 6 werden bei 25°C zusammengegeben und mit einem Schnellmischer 30 Sekunden intensiv miteinander vermischt. Dann wird die Isocyanat-Komponente zugegeben, 5 Sekunden intensiv mit einem Schnellmischer verrührt und sofort ausgegossen.

$T_1$ = 6 Sekunden (Startzeit)
$T_2$ = 62 Sekunden (Steigzeit).

Der Schaum wird 10 Minuten/90°C gehärtet. Er ist geschlossenzellig und weist nach dem Aufbrechen hohe Elastizität auf.

## Beispiel 6

### Herstellung des Carbamats

Vorgelegt werden 27,2 g einer 50%igen eisgekühlten KOH-Lösung (0,243 Mol KOH), 490 g Wasser und 0,5 g Mersolat® H. Innerhalb von 20 Minuten werden 500 g eines 60°C warmen NCO-Präpolymers zugegeben ($OH^{\ominus}$: NCO = 0,6 : 1). Das NCO-Präpolymer wurde aus Toluylendiisocyanat und einem Polypropylenglykolether der durchschnittlichen Molmasse 2000 hergestellt und weist einen NCO-Gehalt von 3,4% auf. Die Innentemperatur wird durch Kühlen im Bereich von 20—24°C gehalten. 1 l Methanol wird zugegeben und 45 Minuten nachgerührt.

## Herstellung des Amins

Obige Reaktionsmischung wird nun mit 290 ml feuchtem Lewatit® SC 108 versetzt. Die bei Raumtemperatur sich nur sehr langsam abspaltende Kohlendioxydmenge verstärkt sich beim Erwärmen auf 55° C. Nach Beendigung der $CO_2$-Entwicklung wird die Reaktionsmischung abgesagt und das beladene Ionenaustauscherharz in 1,5 l Methanol dispergiert und erneut abgesaugt. Beide Filtrate werden vereinigt und die Lösungsmittel bei 20 mbar/100° C und dann 0,15 mbar/80° C abdestilliert (400 g ≧ 82%).

Produktdaten:

NH-Zahl: 10,4 [mg KOH/g]
Molmasse: 10 500
Wassergehalt (Karl Fischer): 14%
Säurezahl: 0,1 [mg KOH/g]
Viskosität ($\eta^{75°C}$): 61 000 (mPa · s).

**Patentansprüche**

1. Verfahren zur Herstellung von Urethan- und/oder Harnstoff- und/oder Biuretgruppen aufweisenden Polyaminen durch Hydrolyse von endständigen Isocyanatgruppen aufweisenden Verbindungen, dadurch gekennzeichnet, daß man
a) in einem ersten Schritt ein Urethan- und/oder Harnstoffgruppen und/oder Biuretgruppen aufweisendes NCO-Präpolymer gegebenenfalls unter Kühlung, durch Vermischen mit wäßrigen oder wäßrige Lösungsmittel enthaltenden Basenlösungen in das Carbamat überführt, wobei das Äquivalentverhältnis zwischen Hydroxid und NCO-Gruppen 0,3 : 1 bis 1,3 : 1 ist,
b) das Carbamat in einem zweiten Schritt durch Umsatz mit einer äquivalenten oder überschüssigen Menge eines sauren Ionenaustauschers unter Abspaltung von Kohlendioxid in das freie Amin überführt,
c) das so erhaltene Polyamin aus dem Reaktionsprodukt auf an sich bekannte Weise abtrennt.

2. Verfahren nach Anspruch 1, dadurch gekennzeichnet, daß als NCO-Präpolymer ein Umsetzungsprodukt aus einem zwei oder drei Hydroxylgruppen aufweisenden Polyether mit einem Molekulargewicht von 400 bis 5000 sowie gegebenenfalls einem Polyol mit einem Molekulargewicht zwischen 62 und 400 und einem Polyisocyanat im NCO/OH-Verhältnis von 1,5 : 1 bis 2,8 : 1 eingesetzt wird.

3. Verfahren nach Anspruch 1, dadurch gekennzeichnet, daß als endständige NCO-Gruppen aufweisende Verbindung ein Umsetzungsprodukt aus einer OH-Verbindung mit einem Molekulargewicht zwischen 18 und 400 und einem Polyisocyanat im NCO/OH-Verhältnis von 1,5 : 1 bis 2,8 : 1 eingesetzt wird.

4. Verfahren nach Ansprüchen 1 bis 3, dadurch gekennzeichnet, daß das NCO-Präpolymer im ersten Verfahrensschritt in Form einer Lösung in einem mit Wasser mischbaren inerten organischen Lösungsmittel eingesetzt wird.

5. Verfahren nach Ansprüchen 1 bis 4, dadurch gekennzeichnet, daß im ersten Verfahrensschritt ein Äquivalentverhältnis zwischen Hydroxylionen und NCO-Gruppen von 1,01 : 1 bis 1,30 : 1 eingehalten wird.

6. Verfahren nach Ansprüchen 1 bis 5, dadurch gekennzeichnet, daß im ersten Verfahrensschritt die Vermischung bei Temperaturen von 0 bis 40° C erfolgt und daß das NCO-Präpolymer in die Basenlösung eingetragen wird.

7. Verfahren nach Ansprüchen 1 bis 6, dadurch gekennzeichnet, daß als Base Alkalihydroxid in Form einer 5- bis 20gew.-%igen Lösung eingesetzt wird.

**Claims**

1. Process for the production of polyamines having urethane and/or urea and/or biuret groups, by the hydrolysis of compounds having terminal isocyanate groups, characterised in that

a) in a first step an NCO prepolymer having urethane and/or urea groups and/or biuret groups is converted into the carbamate by mixing with aqueous solutions of bases or solutions of bases containing aqueous solvents, if appropriate with cooling, the equivalent ratio of hydroxide to NCO groups being 0.3 : 1 to 1.3 : 1,
b) in a second step the carbamate is converted into the free amine by reaction with an equivalent or

excess quantity of an acid ion exchanger, carbon dioxide being split off, and
c) the resulting polyamine is separated from the reaction product in a known manner.

2. Process according to Claim 1, characterised in that the NCO prepolymer used is a reaction product of a polyether having two or three hydroxyl groups and having a molecular weight of 400 to 5,000 and optionally a polyol having a molecular weight of between 62 and 400 and a polyisocyanate in an NCO/OH ratio of 1.5 : 1 to 2.8 : 1.

3. Process according to Claim 1, characterised in that a reaction product from an OH compound having a molecular weight of between 18 and 400 and a polyisocyanate in an NCO/OH ratio of 1.5 : 1 to 2.8 : 1 is used as the compound having terminal NCO groups.

4. Process according to Claims 1 to 3, characterised in that in the first process step the NCO prepolymer is used in the form of a solution in a water-miscible inert organic solvent.

5. Process according to Claims 1 to 4, characterised in that in the first process step an equivalent ratio of 1.01 : 1 to 1.30 : 1 of hydroxyl ions to NCO groups is observed.

6. Process according to Claims 1 to 5, characterised in that in the first process step the mixing is carried out at temperatures of 0 to 40° C and in that the NCO prepolymer is introduced into the solution of the base.

7. Process according to Claims 1 to 6, characterised in that an alkali metal hydroxide in the form of a 5 to 20% by weight solution is used as the base.

**Revendications**

1. Procédé de production de polyamines portant des groupes uréthanne et/ou urée et/ou biuret par hydrolyse de composés présentant des groupes isocyanate terminaux, caractérisé en ce que

a) dans une première étape, un NCO-prépolymère porteur de groupes uréthanne et/ou urée et/ou biuret est transformé en carbamate, le cas échéant avec refroidissement, par mélange avec des solutions basiques aqueuses ou contenant des solvants aqueux, le rapport des équivalents entre hydroxyde et groupes NCO ayant une valeur de 0,3 : 1 à 1,3 : 1,
b) le carbamate est transformé en l'amine libre dans une seconde étape par réaction avec une quantité équivalente ou en exès d'un échangeur ionique acide avec élimination d'anhydride carbonique,
c) la polyamine ainsi obtenue est séparée du produit réactionnel d'une manière connue.

2. Procédé suivant la revendication 1, caractérisé en ce qu'on utilise comme NCO-prépolymère un produit de réaction d'un polyéther porteur de deux ou trois groupes hydroxyle, de poids moléculaire allant de 400 à 5000 ainsi que, le cas échéant, d'un polyol de poids moléculaire compris entre 62 et 400, et d'un polyisocyanate dans le rapport NCO/OH de 1,5 : 1 à 2,8 : 1.

3. Procédé suivant la revendication 1, caractérisé en ce qu'on utilise comme composé porteur de groupes NCO terminaux un produit de réaction d'un composé hydroxylique de poids moléculaire compris entre 18 et 400 et d'un polyisocyanate à rapport NCO/OH allant de 1,5 : 1 à 2,8 : 1.

4. Procédé suivant les revendications 1 à 3, caractérisé en ce que le NCO-prépolymère est utilisé dans la première étape du procédé sous la forme d'une solution dans un solvant organique inerte miscible à l'eau.

5. Procédé suivant les revendications 1 à 4, caractérisé en ce qu'un rapport des équivalents entre les ions hydroxyle et les groupes NCO de 1,0 : 1 à 1,30 : 1 est maintenu dans la première étape du procédé.

6. Procédé suivant les revendications 1 à 5, caractérisé en ce que le mélange est effectué dans la première étape du procédé à des températures de 0 à 40°C et en ce que le NCO-prépolymère est introduit dans la solution basique.

7. Procédé suivant les revendications 1 à 6, caractérisé en ce qu'on utilise comme base un hydroxyde de métal alcalin sous la forme d'une solution à 5—20% en poids.